# EUROPÄISCHE PATENTSCHRIFT

(11) **EP 3 678 618 B1**
(45) Veröffentlichungstag und Bekanntmachung des Hinweises auf die Patenterteilung: **19.04.2023**
(21) Anmeldenummer: 18765848.9
(22) Anmeldetag: 04.09.2018
(51) Int. Cl.: A61F 9/009, B29C 45/00

(54) **FLÜSSIGKEITS-PATIENTENINTERFACE**
LIQUID-PATIENT INTERFACE
INTERFACE PATIENT LIQUIDE

(30) Priorität: 05.09.2017 DE 102017215589
(43) Veröffentlichungstag der Anmeldung: 15.07.2020
(73) Patentinhaber: Carl Zeiss Meditec AG, 07745 Jena (DE)
(72) Erfinder: PÖSSEL, Christian, 99423 Weimar (DE); WOLLWEBER, Thomas, 06648 Eckartsberga (DE); DAMM, Tobias, 81545 München (DE); RILL, Michael Stefan, 07751 Jena (DE); PREUSS, Dirk, 07751 Jena (DE)
(74) Vertreter: Rößner, Ulrike
(86) Internationale Anmeldenummer: PCT/EP2018/073743
(87) Internationale Veröffentlichungsnummer: WO 2019/048428

(56) Entgegenhaltungen:
- JP-A- 2013 248 303
- US-A1- 2016 175 146

## Beschreibung

Die vorliegende Erfindung betrifft ein Flüssigkeits-Patienteninterface zur Fixierung der relativen geometrischen Lage eines Patientenauges zu einem Laser-Applikator eines ophthalmologischen Lasertherapiesystems, das ein Linsenelement und ein Konuselement umfasst, wobei das Linsenelement in das Konuselement eingelegt und dauerhaft so mit dem Konuselement verbunden ist, dass das Flüssigkeits-Patienteninterface einteilig ausgeführt ist. Die Erfindung betrifft weiterhin ein entsprechendes Herstellungsverfahren für ein solches Flüssigkeits-Patienteninterface.

In laserchirurgischen Anwendungen, insbesondere in der lasergestützten Kataraktchirurgie muss die relative geometrische Lage des Patientenauges zum Laserfokus und damit zu dem eingesetzten ophthalmologischen Lasertherapiesystem genau definiert sein. Da bei einer Operation in der Regel Tropfanästhesie verwendet wird, kann der Patient jedoch sein Auge bewusst oder unbewusst bewegen - letztere Bewegungen werden Mikrosakkaden genannt. Um die relative Lage von Auge und Lasertherapiesystem zu fixieren, wird deshalb das Patientenauge mit Hilfe eines Patienteninterfaces (PI), also einer Patientenschnittstelle, an das Lasertherapiesystem mechanisch gekoppelt.

Speziell für die lasergestützte Kataraktchirurgie haben sich dabei sogenannte Flüssigkeits-Patienteninterfaces durchgesetzt. Sie bestehen aus einem trichterförmigen Behälter, der in der Regel mit einer physiologischen Salzlösung (BSS, buffered salt solution) befüllt wird. Des Weiteren enthalten sie ein optisches Element, i.d.R. eine Patienteninterface-Linse, die das distale Ende einer Laseroptik des ophthalmologischen Lasertherapiesystems bildet. Diese Laseroptik fokussiert und leitet den Laserstrahl. Das distale Ende der Laseroptik wird dabei in die Salzlösung getaucht. Diese Anordnung hat Vorteile gegenüber festen Patienteninterfaces, wie z.B. Kontaktgläser: Bei Einsatz von Flüssigkeits-Patienteninterfaces steigt der intraokulare Druck nur geringfügig an. Zudem wird eine korneale Faltenbildung, durch die die optische Abbildung des Laserstrahls in der Augenlinse eines Patientenauges gestört werden könnte, vermieden. Nicht zuletzt erlaubt die Nutzung eines Flüssigkeits-Patienteninterfaces eine Anpassung des Brechungsindex zwischen der Laseroptik und dem Hornhautmaterial des Patientenauges.

Das gesamte Flüssigkeits-Patienteninterface, oder aber zumindest die Teile des Flüssigkeits-Patienteninterfaces, die in direktem Kontakt zum Patientenauge stehen, sind immer steril ausgeführt und müssen für jeden Patienten ausgetauscht werden, da das Auge sehr empfindlich auf bakterielle oder virale Infektionen reagiert. Eine Infektion kann schnell zum Sehverlust führen.

Patienteninterfaces erfüllen also in der lasergestützten Augenchirurgie im Wesentlichen zwei Aufgaben: Sie fixieren das Patientenauge mechanisch zu einem Laser-Applikator eines ophthalmologischen Lasertherapiesystems, durch welchen die Laserstrahlung in das Auge emittiert wird. Zudem sind sie das letzte optische Element einer Laseroptik, insbesondere eines Laserobjektivs. Als entscheidendes Schnittstellenelement müssen sie deshalb sowohl hohe mechanische als auch hohe optische Anforderungen befriedigen.

Derzeit am Markt erhältliche Flüssigkeits-Patienteninterfaces bestehen in der Regel aus mehreren Teilen, die im laufenden OP-Betrieb zusammengestellt werden müssen. Das bedeutet, dass sie vor der Benutzung von der Assistenz oder vom Chirurgen assembliert und/oder beim Ankoppeln an das Patientenauge zusammengeführt werden. Dabei muss stets die Sterilität gewahrt bleiben. Da der Zusammenbau in der Regel nicht einfach ist, kann es deshalb passieren, dass das Flüssigkeits-Patienteninterface vor der Benutzung kontaminiert wird. Die genannten Anforderungen lassen sich mit solchen Flüssigkeits-Patienteninterfaces deshalb nur schwer erfüllen. Beispiele solcher mehrteiliger Flüssigkeits-Patienteninterfaces sind in den Druckschriften US2010/0274228 A1, US 2011/0022035 A1, US2016/0175146 A1 und JP 2013 248303 A beschrieben.

Des Weiteren ist es bei mehrteiligen Flüssigkeits-Patienteninterfaces auch schwierig, die Patienteninterface-Linse reproduzierbar an immer die gleiche Position zu bringen. Da die Patienteninterface-Linse ein wichtiger Teil der Laseroptik ist und eine falsche oder ungenaue Positionierung sich daher direkt auf die optische Qualität des Gesamtsystems auswirkt, ist diese reproduzierbare Positionierbarkeit für den Therapieerfolg essentiell. Viele mechanische Verbindungstechniken, wie beispielsweise Nuten bzw. Federn, Klickverbindungen, Klemmverbindungen, etc., weisen im Gegensatz zu Klebe-, Schweiß- und Angussverbindungen große Abstandtoleranzen zwischen den verbundenen Elementen auf. Deshalb sind vormontierte Flüssigkeits-Patienteninterfaces in der Regel präziser als erst während der Operation zusammensetzbare Flüssigkeits-Patienteninterfaces.

Die WO 2016/058931 A2 beschreibt ein einteiliges Flüssigkeits-Patienteninterface, das sehr einfach, einhändig und ohne Notwendigkeit einer Assistenz vom Chirurgen an das ophthalmologische Lasertherapiesystem koppelbar ist. Eine solche möglichst simple Handhabung eines Flüssigkeits-Patienteninterfaces ist grundsätzlich vorteilhaft. Dann muss aber dafür Sorge getragen werden, dass die Fertigung dieses einteiligen Flüssigkeits-Patienteninterfaces, in der die Montage etwaiger Einzelteile wie der Patienteninterface-Linse mit dem trichterförmigen Behälter bereits vollzogen wird, sehr präzise verläuft. Dies erhöht nun wieder die Komplexität der Fertigung des Flüssig keits-Patienten interfaces.

Aufgabe der vorliegenden Erfindung ist es deshalb, eine Flüssigkeits-Patienteninterface und ein Verfahren zur Herstellung eines solchen Flüssigkeits-Patienteninterfaces zu beschreiben, das konzeptionell möglichst simpel, benutzerfreundlich und effizient einsetzbar ist für den täglichen Gebrauch in der Klinik, gleichzeitig jedoch auch einfach herstellbar ist, so dass die hohen Anforderungen an die technische Präzision sicher erfüllt werden.

Diese Aufgabe wird gelöst durch ein Flüssigkeits-Patienteninterface nach Anspruch 1 sowie durch ein Herstellungsverfahren für ein Flüssigkeits-Patienteninterface nach dem nebengeordneten Anspruch 15.

Vorteilhafte Ausgestaltungen der Erfindung sind in den untergeordneten Ansprüchen beschrieben.

Ein Flüssigkeits-Patienteninterface zur Fixierung der relativen geometrischen Lage eines Patientenauges zu einem Laser-Applikator eines ophthalmologischen Lasertherapiesystems umfasst ein Linsenelement und ein Konuselement. In einem solchen Flüssigkeits-Patienteninterface wird, wie oben beschrieben, eine Flüssigkeit, vorzugsweise BSS, zwischen dem Linsenelement und einem Patientenauge eingefüllt, nachdem das Flüssigkeits-Patienteninterface auf dem Patientenauge aufgesetzt und fixiert worden ist.

Das Linsenelement ist in das Konuselement eingelegt und dauerhaft so mit dem Konuselement verbunden, dass das Flüssigkeits-Patienteninterface einteilig ausgeführt ist.

Dabei wird das Einlegen des Konuselements in das Linsenelement erleichtert, wenn hierfür ein Absatz im Konuselement vorgesehen ist, auf dem das Linsenelement abgelegt werden kann - ein solcher Absatz im Konuselement ist also vorteilhaft. Eine präzise Ablage ist jedoch auch durch alternative Gestaltungen des Linsenelements zum Konuselement möglich. Die Passung zwischen Konuselement und Linsenelement sollte dabei nicht zu eng toleriert werden, da mechanische Kräfte auf das Linsenelement zu undefinierten Änderungen der optischen Eigenschaften führen.

Die relative Lage des Konuselements zum Linsenelement ist aufgrund der erfindungsgemäßen Gestaltung des Flüssigkeits-Patienteninterfaces hingegen unkritisch.

Eine einteilige Ausführung des Flüssigkeits-Patienteninterfaces bedeutet dabei, dass alle Teile des Flüssigkeits-Patienteninterfaces dauerhaft miteinander verbunden werden, so dass sich eine Montage im Operationssaal (OP) vor dem Einsatz des Flüssigkeits-Patienteninterfaces erübrigt, und das Flüssigkeits-Patienteninterface ohne jegliche weitere Montage eingesetzt werden kann, um die relative Lage eines Patientenauges zu einem Laser-Applikator eines ophthalmologischen Lasertherapiesystems zu fixieren. Auf diese Art und Weise ist der Einsatz dieses Flüssigkeits-Patienteninterface auch ohne Unterstützung durch einen Assistenten möglich, und der Konfigurationsaufwand vor einer Operation ist äußerst gering.

Das erfindungsgemäße Flüssigkeits-Patienteninterface ist nun dadurch gekennzeichnet, dass das Linsenelement einstückig ausgebildet ist.

"Einstückig" bedeutet (im Gegensatz zu "einteilig"!), dass das Linsenelement aus einem Stück gefertigt ist.

Das erfindungsgemäße Linsenelement umfasst eine optische Zone, also einen Bereich, durch den im Einsatz der Laserstrahl verläuft. Die optische Zone ist hierfür transparent und enthält eine optische Funktion, die eine Linsenfunktion aufweist. Das Linsenelement umfasst weiterhin einen an die optische Zone anschließenden Mantelbereich, der vorteilhaft ebenfalls leicht konisch ausgebildet ist, mit einer definierten Höhe ungleich Null sowie einem oberen Rand, wobei der obere Rand des Linsenelements eine direkte Verbindung zum Laser-Applikator ermöglicht.

Die Höhe des Mantelbereichs des Linsenelements definiert den effektiven Abstand der letzten Linse des Laser-Applikators des ophthalmologischen Lasertherapiesystems zur oberen Begrenzung bzw. Grenzfläche der optischen Zone des Linsenelements: Dass der obere Rand des Linsenelements eine direkte Verbindung zum Laser-Applikator des ophthalmologischen Lasertherapiesystems ermöglicht, bedeutet, dass dieser obere Rand ohne weitere Zwischenelemente in direkten Kontakt mit dem Laser-Applikator gebracht wird und in Folge eine direkte Verbindung zwischen dem Linsenelement des Flüssigkeits-Patienteninterfaces und dem Laser-Applikator des ophthalmologischen Lasertherapiesystems aufgebaut wird. Durch den in der Regel leicht konisch ausgebildeten Mantelbereich und den sich anschließenden oberen Rand wird das Bildfeld durch die Verbindung zum Laser-Applikator nicht eingeschränkt.

Damit ist das Linsenelement eingerichtet, die relative geometrische Lage eines letzten optischen Applikator-Elements des Laser-Applikators des ophthalmologischen Lasertherapiesystems (d.h., i.d.R. einer Linse) zur optischen Zone des Linsenelements präzise und wiederholbar zu definieren.

Dadurch ist es wiederum möglich, die relative geometrische Lage eines in einem ophthalmologischen Lasertherapiesystem erzeugten Laserfokus zu einem Patientenauge präzise und wiederholbar zu bestimmen. "Präzise" bedeutet hier mit im Vergleich zum Stand der Technik deutlich geringeren Abweichungen, da im Vergleich zum Stand der Technik keinerlei Zwischenelemente, denen wiederum eine Fertigungstoleranz zugestanden werden müsste, zum Einsatz kommen. "Wiederholbar" ist die Positionierung der Lage des Laserfokus zu einem Patientenauge, weil die Positionierung der optischen Zone des Linsenelements des Flüssigkeits-Patienteninterfaces zum letzten optischen Element des Laser-Applikators wiederum aufgrund der direkten Fixierung des Linsenelements am Laser-Applikator in immer gleicher Weise erfolgen kann. Damit ist das den Laserfokus in seiner Struktur und Lage bestimmende optische Gesamtsystem präzise und wiederholbar definiert. Der Abstand der optischen Zone des Linsenelements des Flüssigkeits-Patienteninterfaces zum Patientenauge ist hingegen unkritisch, da dieser während der OP-Planung individuell festgelegt wird, beispielsweise über eine OCT (= "optische Kohärenztomographie") -Bildgebung.

Das mechanische Interface für den direkten Kontakt und die direkte Verbindung vom Linsenelement zum Laser-Applikator sowie die Höhe h des Mantelbereichs des Linsenelements sind folglich kritisch bei diesem Flüssigkeits-Patienteninterface-Design. Diese direkte Verbindung des Linsenelements mit dem Laser-Applikator ist jedoch konstruktiv von Vorteil, da man damit alle kritischen Anforderungen an die Fertigungsgenauigkeit, d.h., an optische und mechanische Parameter nur in dieser einen Teilkomponente - dem Linsenelement - adressiert. Würde man das Flüssigkeits-Patienteninterface hingegen über das Konuselement an den Laser-Applikator ankoppeln, müsste man das Linsenelement wiederum mechanisch definiert und mit sehr hoher Präzision mit dem Konuselement verbinden. Man hätte dann eine zusätzliche Schnittstelle mit sehr engen Toleranzgrenzen. Diese wird durch das erfindungsgemäße Design jedoch vermieden: Alle kritischen Parameter liegen im Linsenelement selbst.

Da die Genauigkeitsanforderungen an die laterale und axiale Position der optischen Zone des Flüssigkeits-Patienteninterfaces relativ zur Laseroptik des ophthalmologischen Lasertherapiesystems über das Linsenelement abgedeckt sind, sind die Fertigungstoleranzen für das Konuselement unkritisch. Wie schon oben begründet, ist auch der Abstand des Linsenelements zum Patientenauge unkritisch. Dadurch ist die relative Lage des Konuselements zum Linsenelement unkritisch, denn sämtliche kritischen Anforderungen sind im einteiligen Linsenelement vereint.

Der hier dargestellte erfindungsgemäße Aufbau des Flüssigkeits-Patienteninterfaces erlaubt damit auch einen sehr unkomplizierten Therapie-Workflow:
Im Therapie-Workflow wird das Flüssigkeits-Patienteninterface zunächst an den Laser-Applikator des ophthalmologischen Lasertherapiesystems angebracht. Danach wird das an den Laser-Applikator gekoppelte Flüssigkeits-Patienteninterface direkt auf das Patientenauge aufgesetzt und mittels Vakuum-Ansaugung über eine Ansauglippe, die am unteren Rand des PI-Konus angeordnet ist, angesaugt. Der Zwischenraum zwischen dem Patientenauge und dem Linsenelement wird dann mit einer biokompatiblen Flüssigkeit befüllt, um den Brechungsindex-Unterschied zu nivellieren und damit Rückreflexe zu reduzieren und die Transmission zu erhöhen, und andererseits das Patientenauge während der Prozedur nicht austrocknen zu lassen.

Eine besondere Ausführungsform des erfindungsgemäßen Flüssigkeits-Patienteninterface ist dadurch gekennzeichnet, dass der obere Rand des Linsenelements eine Struktur zur Ausbildung einer mechanisch stabilen, direkten Verbindung mit einer Applikator-Schnittstelle des Laser-Applikators des ophthalmologischen Lasertherapiesystems aufweist.

Das mechanische Interface, also die Grenzfläche, für den direkten Kontakt und die direkte Verbindung vom Linsenelement zum Laser-Applikator, und insbesondere zu einer Applikator-Schnittstelle des Laser-Applikators, wird gebildet durch den oberen Rand des Linsenelements. Dieser weist eine entsprechende Struktur zur Ausbildung einer direkten Verbindung mit der Applikator-Schnittstelle auf, deren Ausprägung und Fertigungspräzision kritisch ist für diesen Flüssigkeits-Patienteninterface-Aufbau. Dafür fallen jedoch kritische Anforderungen an das Konuselement weg, wie sie im Stand der Technik üblich sind.

In verschiedenen vorteilhaften Ausführungsformen ist das Linsenelement des erfindungsgemäßen Flüssigkeits-Patienteninterfaces, und insbesondere die Struktur seines oberen Randes, in unterschiedlicher Art und Weise ausgebildet, um eine mechanisch stabile, direkte Verbindung mit einer Applikator-Schnittstelle des Laser-Applikators des ophthalmologischen Lasertherapiesystems zu realisieren.

In einer ersten Alternative umfasst die Struktur des oberen Randes des Linsenelements eine Stirnfläche zur Ausbildung einer vakuumdichten Verbindung mittels Vakuum-Ansaugung zu der Applikator-Schnittstelle, wobei die Vakuum-Ansaugung über die Evakuierung eines Volumens erfolgt, das begrenzt wird von einem letzten optischen Applikator-Element, der Applikator-Schnittstelle und dem Linsenelement, und die Applikator-Schnittstelle hierfür einen Vakuumansaugkanal in das Volumen aufweist.

In einer zweiten Alternative umfasst die Struktur des oberen Randes des Linsenelements eine Stirnfläche zur Ausbildung einer vakuumdichten Verbindung mittels Vakuum-Ansaugung zu der Applikator-Schnittstelle, wobei die Vakuum-Ansaugung über die Stirnfläche erfolgt, und die Applikator-Schnittstelle hierfür einen Vakuumansaugkanal aufweist, der auf die Stirnfläche aufsetzt. Der Vakuumansaugkanal sollte in diesem Fall bevorzugt über einen großflächigen Bereich der Stirnfläche ansaugen können.

Für diese beiden Alternativen muss die Stirnfläche bündig an die Applikator-Schnittstelle anschließen. Dies ist beispielsweise realisiert, wenn die Stirnfläche planparallel zur Applikator-Schnittstelle ausgeführt ist und glatt poliert ist.

In einer dritten Alternative weist der obere Rand des Linsenelements eine Struktur auf, die eine formschlüssige und/oder kraftschlüssige Verbindung mit der Applikator-Schnittstelle ermöglicht. Formschlüssige Verbindungen entstehen durch das Ineinandergreifen von mindestens zwei Verbindungspartnern. Dadurch können sich die Verbindungspartner auch ohne oder bei unterbrochener Kraftübertragung nicht lösen. Anders ausgedrückt ist bei einer formschlüssigen Verbindung der eine Verbindungspartner dem anderen im Weg. Kraftschlüssige Verbindungen setzen eine Normal-Kraft auf die miteinander zu verbindenden Flächen voraus. Ihre gegenseitige Verschiebung ist verhindert, solange die durch die Haftreibung bewirkte Gegen-Kraft nicht überschritten wird.

Es muss jedoch stets gewährleistet sein, dass sich die optischen Eigenschaften des Linsenelements, und insbesondere der optischen Zone des Linsenelements, durch diese Verbindung mit der Applikator-Schnittstelle des Laser-Applikators nicht ändern. Hierfür muss insbesondere jegliche Verspannung im Verlauf des Aufbaus dieser Verbindung vermieden werden.

Im Falle des Flüssigkeits-Patienteninterfaces, bei dem erfindungsgemäß eine Verbindung über die Struktur des oberen Randes des Linsenelements erzielt wird, ist zudem eine Verbindung von Vorteil, die sofort schließt und mechanisch keinen Abrieb erzeugt. Beispiele hierfür sind eine "Klickverbindung" mit der Applikator-Schnittstelle, d.h., das Flüssigkeits-Patienteninterface und insbesondere das Linsenelement des Flüssigkeits-Interfaces wird auf die Applikator-Schnittstelle aufgesetzt und durch kurze mechanische Bewegung, bei der eine Struktur der Applikator-Schnittstelle und eine Struktur des oberen Randes des Linsenelements ineinander einrasten, in eine fixierte Stellung gebracht. Diese Verbindung basiert also auf einem Schlüssel-Schloss-Prinzip und die Struktur der einen Seite - also der Applikator-Schnittstelle - definiert damit die Struktur der anderen Seite - also des oberen Randes des Linsenelements -, oder umgekehrt. Auch denkbar sind Klemmverbindungen, sofern nur ein für die optische Zone des Linsenelements vernachlässigbarer Druck ausgeübt wird.

In einer besonderen Ausführungsform ist das erfindungsgemäße Flüssigkeits-Patienteninterface des Weiteren dadurch gekennzeichnet, dass der obere Rand des Linsenelements eine positive Ausrichtstruktur aufweist, bevorzugt einen Absatz, die eingerichtet ist, in eine an der Applikator-Schnittstelle angeordnete negative Ausrichtstruktur einzugreifen.

Es ist von Vorteil, wenn das Linsenelement des Flüssigkeits-Patienteninterfaces eine zusätzliche seitliche (d.h. laterale) Ausrichtung ermöglicht, um das Flüssigkeits-Patienteninterface und insbesondere das Linsenelement auch lateral hochpräzise auf die Applikator-Schnittstelle und letztlich die Apertur des Laser-Applikators ausrichten zu können. Dies wird durch die beschriebene Ausrichtstruktur gewährleistet.

Eine entsprechende negative Ausrichtstruktur an der Applikator-Schnittstelle zu einem Absatz im oberen Rand des Linsenelements ist beispielsweise durch einen oder mehrere Zapfen oder einen zum Absatz des oberen Randes des Linsenelements passenden negativer Absatz realisiert.

Von Vorteil ist, wenn das Linsenelement des erfindungsgemäßen Flüssigkeits-Patienteninterfaces aus einem Polymer, bevorzugt aus Polycarbonat, oder aus optischem Glas, bevorzugt aus Siliziumdioxid, besteht. Das optische Material des Linsenelements muss eine hohe Transparenz für die Therapiewellenlängen und die Untersuchungswellenlängen (z.B. OCT-Wellenlängen) bieten. Wird es in der refraktiven Chirurgie oder - ganz besonders bevorzugt - in der Kataraktchirurgie eingesetzt, so wird üblicherweise eine gepulste Laserstrahlung, typischerweise aus einer Femtosekunden-Laserquelle oder einer Pikosekunden-Laserquelle, als Therapielaserstrahlung genutzt. Typische Untersuchungswellenlängen sind wiederum alle in der optischen Kohärenztomographie genutzten Wellenlängen, aber auch die Wellenlängen von sichtbarem Licht und Infrarot-Strahlung.

Linsenelemente aus Polymeren haben den Vorteil, dass sie reproduzierbar mittels Spritzgussverfahren in sehr hohen Stückzahlen gefertigt werden können. Die Herstellkosten sind in diesem Fall wesentlich geringer als bei Glaslinsen. Hingegen lassen sich Glaslinsen mit gängigen Verfahren präziser herstellen, während Polymerlinsen zu höheren Toleranzabweichungen führen.

Polymerlinsen können in großen Stückzahlen und zu günstigen Preisen z.B. durch ein Spritzgussverfahren hergestellt werden. Dabei wird die abzugießende Geometrieform als Negativ in eine Gussform gebracht. Diese Gussform besteht in der Regel aus chemisch stabilen und formstabilen Materialien wie z.B. Stähle oder technische Keramiken, die viele Abgüsse erlauben und dabei stets die Anforderungen an die gegebenen Toleranzen erfüllen. Beim Spritzgussverfahren wird zunächst das Polymergranulat thermisch verflüssigt und durch einen Heißkanal in die Spritzgussform unter hohem Druck (ca. 500 bis 2000 bar) gepresst. Der Ort, an dem die Polymerschmelze in die Gussform dringt, nennt man Angusspunkt. Ausgehend vom Angusspunkt verteilt sich das flüssige Polymer in der Form. Durch eine Rückstromsperre wird ein Zurückströmen der Polymerschmelze in Richtung Angusspunkt verhindert. Während des Einspritzens wird versucht, ein möglichst laminares Fließverhalten der Schmelze zu erreichen, d.h. die Schmelze wird am Gussformrand sofort abgekühlt und erstarrt. Die nachrückende Schmelze wird durch den dadurch verjüngten Schmelzkanal mit noch höherer Geschwindigkeit gedrückt und vorne an der Schmelzfront zum Rand hin dehndeformiert. Das Abkühlen der Polymerschmelze führt in der Regel zu einer Volumenschwindung, die sich auf Maßhaltigkeit und Oberflächenqualität des Gusses auswirkt.

Eine Fertigung von Linsenelementen aus Polymeren im Spritzgussverfahren ist aus diesen Gründen im Übrigen auch für die Herstellung des klassischen sogenannten "Kontaktglases", also eines Patienteninterfaces bzw. einer Kontaktvorrichtung, bei dem die optisch aktive Zone des Linsenelements direkt auf die Hornhaut des Patientenauges aufgesetzt wird und also keine Befüllung mit einer physiologischen Salzlösung nötig ist, von großem Vorteil. Solche Kontaktgläser werden üblicherweise in der refraktiven Laserchirurgie bei der Bearbeitung der Hornhaut eingesetzt - also dort, wie der Laserstrahl, beispielsweise ein Femtosekunden-Laserstrahl, nicht so tief in die Augenstrukturen eindringen muss, sondern oberflächennah gearbeitet wird. Auch in diesem Fall ist ein definierter Abstand der optischen Zone des Linsenelements zum ersten optischen Element eines Laser-Applikators eines ophthalmologischen Lasertherapiesystems sehr wichtig. Die Präzision der Ausführung des Linsenelements selbst ist hier sogar von noch größerer Wichtigkeit: Die Oberflächengenauigkeit muss sehr hoch und die Brechungsindex-Verteilung über das gesamte Linsenelement muss absolut stabil sein.

Linsenelemente eines klassischen Kontaktglases benötigen aufgrund der wesentlich geringeren Eindringtiefe des fokussierten Laserstrahls in der Regel keinen an die optische Zone anschließenden (konischen) Mantelbereich mit einer definierten Höhe ungleich Null bzw. keinen separaten oberen Rand, um eine direkte Verbindung zum Laser-Applikator zu ermöglichen und trotzdem einen Durchmesser des Bildfelds zu erhalten, der so weit ist, dass ein problemloses Arbeiten, insbesondere in der gesamten Kornea eines Patientenauges, möglich ist.

Durch die Nutzung eines Spritzgussverfahrens sind also erfindungsgemäß ebenfalls ein Kontaktglas und ein Verfahren zur Herstellung eines solchen Kontaktglases, das konzeptionell simpel, benutzerfreundlich und effizient einsetzbar ist für den täglichen Gebrauch in der Klinik, gleichzeitig jedoch auch einfach herstellbar ist, so dass die hohen Anforderungen an die technische Präzision sicher erfüllt werden, folgendermaßen beschrieben:
Kontaktglas zur Fixierung der relativen geometrischen Lage eines Patientenauges zu einem Laser-Applikator eines ophthalmologischen Lasertherapiesystems, umfassend ein Linsenelement und ein Konuselement, hier auch Kontaktglashalter genannt,
wobei das Linsenelement in das Konuselement eingelegt und dauerhaft so mit dem Konuselement verbunden ist, dass das Kontaktglas einteilig ausgeführt ist, und
wobei das Linsenelement
   - einstückig ausgebildet ist, eine optische Zone mit einer Linsenfunktion aufweist, die vorzugsweise über den Durchmesser des Linsenelements verläuft,
   - vorzugsweise augenseitig an die Krümmung der Hornhaut angepasst und auf der dem Auge abgewandten Seite planar ausgeführt ist,
   - einen umlaufenden Randbereich mit einer konstante Dicke ungleich Null aufweist, dadurch gekennzeichnet, dass das Linsenelement aus einem Polymer durch ein Spritzgussverfahren ausgebildet ist.

Dabei umfasst das Konuselement in diesem Fall nur eine Wandung, eine untere Sauglippe und eine Vakuum-Durchführung, denn bei der Anwendung des Kontaktglases zum Fixieren der Position des Auges zum Laser-Applikator des ophthalmologischen Lasertherapiesystems befindet sich das Linsenelement direkt auf der Hornhaut des Patientenauges. Eine Befüllung mit einer physiologischen Salzlösung ist hier nicht nötig.

Damit ist auch Teil der erfindungsgemäßen Lösung ein Patienteninterface bzw. eine Kontaktvorrichtung zur Fixierung der relativen geometrischen Lage eines Patientenauges zu einem Laser-Applikator eines ophthalmologischen Lasertherapiesystems, das als Kontaktglas oder als Flüssigkeits-Patienteninterface ausgestaltet ist, umfassend ein Linsenelement und ein Konuselement, wobei das Linsenelement in das Konuselement eingelegt und dauerhaft so mit dem Konuselement verbunden ist, dass das Kontaktglas einteilig ausgeführt ist, und wobei das Linsenelement
- einstückig ausgebildet ist,
- eine optische Zone mit einer Linsenfunktion aufweist,
- eine definierte Höhe ungleich Null aufweist, die den Abstand des letzten optischen Elements des Laser-Applikators des ophthalmologischen Lasertherapiesystems zum Austrittsort des Laserstrahls aus dem optischen Zone des Linsenelements präzise bestimmt,
- dadurch gekennzeichnet, dass das Linsenelement zudem einen Randbereich aufweist, der eine direkte Verbindung zum Laser-Applikator ermöglicht und aus einem Polymer durch ein Spritzgussverfahren ausgebildet ist.

Durch eine solche Lösung gelingt die Herstellung eines Patienteninterfaces - sowohl eines Kontaktglases als auch eines Flüssigkeits-Patienteninterfaces - in hoher Präzision, da in beiden Varianten das Linsenelement das einzige Element des Patienteninterfaces ist, das in höchster Maßhaltigkeit und optischer Qualität produziert werden muss, weil es alle kritischen Anforderungen an das Patienteninterface auf sich vereint. Das Konuselement, in das das Linsenelement dann aufgenommen wird, wie auch der Prozess des Zusammenfügens der beiden Elemente können hingegen wesentlich höhere Fertigungstoleranzen aufweisen. Durch die Herstellung des Linsenelements im Spitzgussverfahren ist eine einfache und hochvolumige Herstellung dieses Linsenelements möglich.

Ist das Linsenelement des Flüssigkeits-Patienteninterfaces oder eines Kontaktglases aus einem Polymer durch ein Spritzgussverfahren ausgebildet, so ist es von Vorteil, wenn der Angusspunkt außerhalb der optischen Zone angeordnet ist, da es sonst zu Einschränkungen des nutzbaren Bereiches der optischen Zone käme.

Vorteilhaft ist es, wenn der Angusspunkt eines durch ein Spritzgussverfahren ausgebildeten Linsenelements des Flüssigkeits-Patienteninterfaces in einer ersten Alternative an einer Seite des oberen Randes des Linsenelements, an dem dieses einen maximalen Durchmesser aufweist, angeordnet ist, wobei diese Seite derart abgeschrägt wird, dass ein beim Angießen entstehender Zapfen den maximalen Durchmesser des oberen Randes nicht überschreitet. Würde der Angusspunkt den maximalen Durchmesser überschreiten, dann klemmte das Linsenelement bei der Montage in dem Konuselement, was die optischen Eigenschaften der optischen Zone des Linsenelements undefiniert verändern kann.

Insbesondere ist aber darauf zu achten, dass der Angusspunkt nicht die direkte Verbindung des Linsenelements mit der Applikator-Schnittstelle des Laser-Applikators behindert: Er sollte also beispielsweise nicht die Struktur des oberen Randes stören, insbesondere nicht, wenn eine Vakuum-Ansaugung über eine Stirnfläche des oberen Randes vorgesehen ist.

In einer zweiten Alternative ist der Angusspunkt eines durch ein Spritzgussverfahren ausgebildeten Linsenelements an der Innenseite des oberen Randes oder an der Außenseite des Mantelbereiches, insbesondere an der Außenseite des unteren Abschnitts des Mantelbereichs, angeordnet.

Die Wandstärken des Linsenelements können grundsätzlich beliebig gewählt werden. Im Falle von spritzgegossenen Linsenelementen eignen sich besonders Konstruktionen mit nahezu konstanter Wandstärke, da sich in diesem Fall die flüssige Polymerschmelze gleichmäßiger verteilt und Schrumpfungen des Materials beim Kühlen besser vorkompensiert werden können. Durch gleichmäßiges Schrumpfen werden die optischen Eigenschaften berechenbar.

Bevorzugt ist die untere Begrenzungsfläche der optischen Zone des Linsenelements des Flüssigkeits-Patienteninterfaces, also die dem Patientenauge zugewandte Fläche, als optisches Element des Flüssigkeits-Patienteninterfaces ausgebildet und weist insbesondere eine Linsenfunktion auf. Damit ist sie als letztes optisches Element für die Definition des Laserfokus in seiner Struktur und Lage mitverantwortlich. Wenn das Linsenelement mit der Applikator-Schnittstelle des Laser-Applikators eines ophthalmologischen Lasertherapiesystems verbunden wird, ist die optische Zone des Linsenelements somit Teil eines optischen Gesamtsystems zur Formung und Positionierung des Laserfokus. Durch die Möglichkeit der präzisen und wiederholbaren Positionierung trägt sie somit auch zur qualitativ hochwertigen Formung und Positionierung des Laserfokus bei.

Von besonderem Vorteil ist ein Linsenelement des Flüssigkeits-Patienteninterfaces, dessen obere Begrenzungsfläche der optischen Zone, also die dem Patientenauge abgewandte Seite, eine Antireflexschicht bzw. ein Antireflexschichtsystem aufweist.

Eine Antireflexschicht reduziert die Reflexionen an der Grenzfläche des Linsenelements zur Luft bzw. zum Vakuum, die entstehen würden, weil hier gewöhnlich eine große Differenz der Brechungsindices zwischen Luft bzw. Vakuum und dem Material des Linsenelements vorliegt. Sie vermeidet somit Rückreflexionen
- beispielsweise der hochenergetischen Laserstrahlung - in den Laser-Applikator, welche interne Komponenten, wie beispielsweise Linsen, Prismen, Detektoren, etc. zerstören könnten. Dabei wird sehr häufig nicht nur mit einer isolierten Antireflexschicht gearbeitet, sondern ein Antireflexschichtsystem eingesetzt. Im vorliegenden Fall hat ein solches Antireflexschichtsystem eine frei wählbare bzw. den Notwendigkeiten angepasste Schichtfolge.

Wird eine Antireflexschicht bzw. ein Antireflexschichtsystem auf der oberen Begrenzungsfläche des Linsenelements des erfindungsgemäßen Flüssigkeits-Patienteninterfaces genutzt, dann ist sie so ausgebildet, dass die Reflexion einer Strahlung in mindestens einem der folgenden Wellenlängenbereiche unterbunden wird, wobei ein Unterbinden im jeweiligen Wellenlängenbereich mit einer zum Wellenlängenbereich angegebenen Reflexion R erfolgt:
- Für die Therapielaserstrahlung Wellenlängen von 1000nm bis 1100nm, mit einer Reflexion R kleiner 1%;
- Für die OCT-Laserstrahlung Wellenlängen von 800nm bis 1200nm, mit einer Reflexion R kleiner 1%;
- Für die Nutzung von Infrarotlicht (beim Einsatz von Infrarot-Kameras) Wellenlängen von 800nm bis 1000nm, mit einer Reflexion R kleiner 10%;
- Für die Nutzung von Licht im sichtbaren Bereich, insbesondere wenn das Flüssigkeits-Patenteninterface genutzt wird in Verbindung mit einem Lasertherapiesystem, das ein Operationsmikroskop verwendet, dessen Operationsmikroskop-Kopf mit einem Laser-Applikator koppelbar ist, so dass allein schon dadurch sichtbares Licht durch das Flüssigkeits-Patienteninterface auf das Auge des Patienten fällt, Wellenlängen von 400nm bis 700nm, mit einer über diesen Wellenlängenbereich möglichst konstant bleibenden Reflexion R, d.h., ohne signifikante Minima oder Maxima, so dass keine signifikanten Änderungen der Absorptionen bzw. Reflexionen im sichtbaren Bereich auftreten, und beim Durchblick durch die optische Zone des Linsenelements keine Farbverfälschungen entstehen.

Da häufig eine Kombination der genannten Strahlungen zur Untersuchung und zur Therapie eingesetzt wird, muss die Antireflexschicht bzw. das Antireflexschichtsystem derart gestaltet sein, dass Licht aus verschiedenen genutzten und oben angegebenen Spektralbereichen so wenig wie möglich reflektiert wird. Wie schon erwähnt, ist die konkrete Schichtfolge dieser Antireflex-Beschichtung, also sowohl das Material als auch die Dicke, frei so wählbar, dass eine Reflexion im kritischen Wellenlängenbereich bzw. in den kritischen Wellenlängenbereichen (siehe oben) unterbunden bzw. wie oben angegeben minimiert wird. Die Wahl der optimalen Schichtmaterialien hängt jedoch auch stark vom Material des Linsenelements selbst ab, da Aspekte wie Haftfestigkeit, Biokompatibilität, Abstimmung auf die Laserwellenlänge, etc. im gegebenen medizinischen Umfeld beachtet werden müssen.

Weiterhin ist es von Vorteil, wenn das Linsenelement des Flüssigkeits-Patienteninterfaces eingerichtet ist, an einer Stirnseite des oberen Randes, die dazu vorzugsweise poliert ist, eine aus der Applikator-Schnittstelle auskoppelnde Beleuchtung aufzunehmen, und/oder wenn
- in einer alternativen bzw. parallel nutzbaren Variante - das Konuselement eingerichtet ist, lokal einen direkten Kontakt zur Applikator-Schnittstelle auszubilden, wobei diese mechanische Koppelstelle ebenfalls poliert sein sollte, um hierüber eine aus dem Laser-Applikator auskoppelnde Beleuchtung aufzunehmen.

In einer Konfiguration eines ophthalmologischen Lasertherapiesystems wird der Laser-Applikator zusammen mit einem Operationsmikroskop (OPMI) verwendet. In einer Variante führt in diesem Fall der Beobachtungspfad vom des Operationsmikroskops durch die Optik des Laser-Applikators hindurch bis hin zum Patientenauge. In der Regel wird der Beleuchtungspfad des Operationsmikroskops dabei koaxial zum oder nahe am Beobachtungspfad geführt. In diesem Fall durchquert der Beleuchtungspfad auch die optische Zone des Linsenelements, was wiederum zu störenden Reflexionen im Bild führen kann. In alternativen bzw. ggf. auch parallel nutzbaren Varianten kann man nun in diesem Fall die Beleuchtung des Patientenauges so gestalten, dass sie seitlich in das Linsenelement an einem Ort einkoppelt, der nicht zur optischen Zone gehört, oder aber dass die Beleuchtung des Auges über das Konuselement erfolgt.

Eine weitere Ausführungsform des erfindungsgemäßen Flüssigkeits-Patienteninterface ist durch ein Konuselement gekennzeichnet, das eine Konuswandung, eine untere Sauglippe, eine Vakuum-Durchführung, und einen Einfüllkanal für Flüssigkeiten umfasst, wobei die Vakuum-Durchführung durch die Konuswandung in die Sauglippe verläuft, und der Einfüllkanal für Flüssigkeiten durch die Konuswandung in ein bei der Vakuum-Ansaugung von einem Patientenauge, der Konuswandung und dem Linsenelement gebildetes zweites Volumen verläuft.

Zur Fixierung des Flüssigkeits-Patienteninterfaces wird dann die Vakuum-Durchführung mit einem Schlauch an eine Vakuumpumpe angeschlossen, während der Einfüllkanal mit einem entsprechenden Flüssigkeitsreservoir verbunden wird.

Vorteilhaft ist es auch, wenn das Linsenelement mit dem Konuselement des Flüssigkeits-Patienteninterfaces mit Hilfe eines Klebers verklebt ist, der beim Trocknungsvorgang keinerlei Zug- oder Verspannkräfte ausübt. Durch derartige auftretenden Kräfte würde das Linsenelement verformt und änderte so seine optischen Eigenschaften auf undefinierte Weise, so dass Passe (also Passformfehler) und/oder Astigmatismus entstehen könnten. Der Kleber und alle den Patienten direkt oder indirekt berührenden Komponenten müssen zudem biokompatibel sein.

Besonders günstig ist es zudem, wenn das Konuselement des Flüssigkeits-Patienteninterfaces einen Kragen umfasst, der als Verlängerung der Konuswandung ausgebildet ist. Dadurch wird nach Befestigung des Flüssigkeits-Patienteninterfaces am Laser-Applikator eines ophthalmologischen Lasertherapiesystems der Sterilbereich des Laser-Applikators, insbesondere im kritischen Teil nahe des Patientenauges, vergrößert: Der Kragen geht über die Applikator-Schnittstelle hinaus und hüllt diese bzw. den gesamten unteren Teil des Laser-Applikators bis zu einer durch eine Kragenhöhe festgelegten Höhe ein.

Der Kragen des Konuselements schmiegt sich dabei bei Befestigung des Flüssigkeits-Patienteninterfaces am Laser-Applikator in einer bevorzugten Ausführung nicht direkt am Laser-Applikator an, sondern lässt einen kleinen Spalt, so dass ggf. ein Drape zwischen dem Laser-Applikator und dem Flüssigkeits-Patienteninterface eingebracht werden kann.

Besonders vorteilhaft für eine sichere Befüllung des Flüssigkeits-Patienteninterfaces ist es, wenn der Einfüllkanal im Konuselement so gegenüber der Vakuum-Durchführung versetzt angeordnet ist, dass der Einfüllkanal und die Vakuum-Durchführung in einer Ansicht von oben auf das Flüssigkeits-Patienteninterface nicht übereinander fallen.

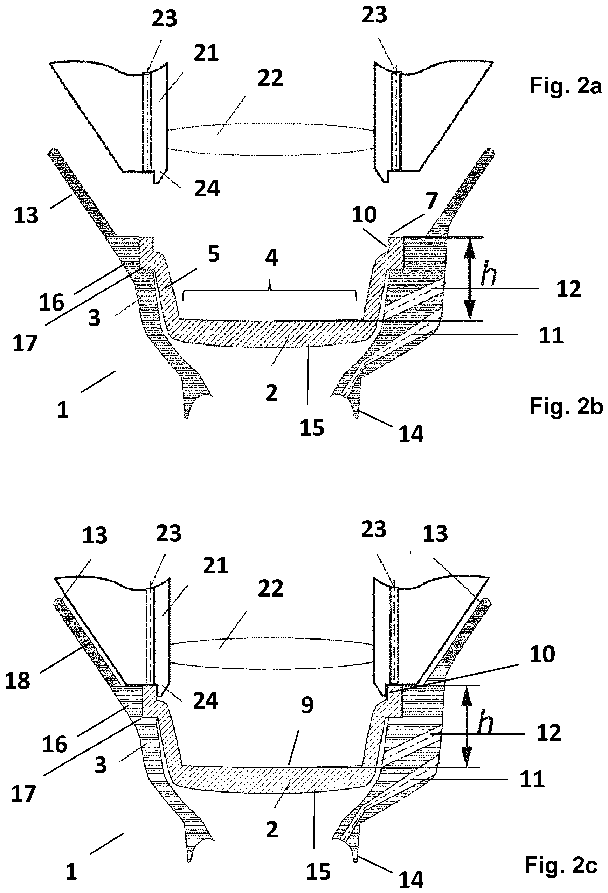

Wie aus den Fig. 2b und 2c ersichtlich, kann der Abstand zwischen dem Konuselement 3 und dem Linsenelement 2 an der üblichen Position des Einfüllkanals 12 für Flüssigkeiten aus konstruktiven Gründen sehr gering werden. Nach dem Stand der Technik wurden der Einfüllkanal 12 und die Vakuum-Durchführung 11 bislang übereinander angeordnet, da dies erlaubt, die Zuführungsschläuche zu diesem Kanälen 11, 12 gemeinsam zu führen, die in der Regel ebenfalls am Flüssigkeits-Patienteninterface 1 vormontiert sind, so dass sie nur noch an eine Vakuum-Pumpe bzw. ein Flüssigkeitsreservoir angeschlossen werden müssen. Würden diese an völlig unterschiedlichen Seiten durch die Konuswandung geführt, so würde sich die Fixierung des Flüssigkeits-Patienteninterfaces aufgrund der einzelnen herabhängenden Schläuche wiederum schwieriger gestalten. Jedoch soll einerseits der Außendurchmesser des Konuselements 3 möglichst klein sein, so dass das Flüssigkeits-Patienteninterface bei möglichst vielen Patienten genutzt werden kann, d.h., auch bei kleinen und tiefliegenden Augen. Andererseits soll die optische Zone 4 des Linsenelements 2 möglichst groß sein, um einen möglichst großen optisch wirksamen Bereich für die Therapie zur Verfügung zu haben. Daraus entsteht das Problem, dass es bei Flüssigkeiten mit hoher Viskosität passieren kann, dass die Flüssigkeit nicht in das Volumen zwischen dem Linsenelement 2 und dem Patientenauge fließt, sondern durch die Oberflächenspannung seitlich abfließt.

Man muss also versuchen, den Abstand zwischen dem Konuselement 3 und dem Linsenelement 2 so groß wie möglich zu gestalten. Deshalb muss der Einfüllkanal 12 im Konuselement möglichst weit nach unten (also in Richtung Patientenauge) gezogen werden, so dass die Befüllung des Flüssigkeits-Patienteninterfaces 1 mit der Flüssigkeit durch einen Einfüllkanal 12 erfolgt, der im Idealfall unterhalb unteren Grenzfläche der optischen Zone 4 des Linsenelements 2 angeordnet ist, dessen Lage sich aber in der Realität zumindest diesem Idealfall nähert. Wenn jedoch das Flüssigkeits-Patienteninterface 1 mittels Vakuum an das Patientenauge 50 gekoppelt wird, kommt es hier zu einer konstruktiven Kollision. Die beiden Kanäle 11 und 12 dürfen sich anwendungsbedingt nicht kreuzen oder überschneiden.

Eine zueinander versetzte Anordnung von Einfüllkanal für die Flüssigkeit, beispielsweise BSS, und der Vakuum-Durchführung, derart dass beide Kanäle nach wie vor in der Nähe zueinander angeordnet sind, jedoch in einer Ansicht von oben nicht mehr übereinander fallen, ermöglicht es, die Zuführungsschläuche nach wie vor gemeinsam zu führen, aber den Einfüllkanal 12 durch die Konuswandung für die Flüssigkeitsbefüllung in einen Bereich des Konuselements 3 - weiter nach unten - zu bringen, in dem der Abstand zwischen Konuselement 3 und Linsenelement 2 deutlich größer ist, als wenn der Einfüllkanal 12 - wie vorher üblich - über der Vakuum-Durchführung 11 angeordnet ist.

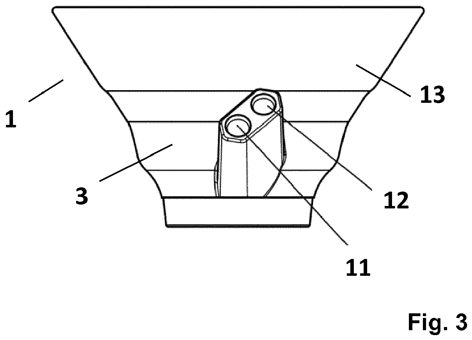

Der Einfüllkanal 12 wird also gegenüber der Vakuum-Durchführung 11, wie in der Fig. 3 gezeigt, versetzt und nach unten verlegt. In diesem Fall können auch zähflüssige Fluide eingefüllt werden.

Das erfindungsgemäße Flüssigkeits-Patienteninterface ist also aufgrund seiner speziellen hier beschriebenen Merkmale simpel, benutzerfreundlich und effizient einsetzbar für den täglichen Gebrauch in der Klinik und ermöglicht das Handling während der OP durch eine einzelne Person (also des Chirurgen oder eines Assistenten). Zudem ist es bezüglich seiner Herstellbarkeit und seines zuverlässigen Einsatzes optimiert auf eine hohe technische Präzision, die durch das Beherrschen im Vergleich zu Flüssigkeits-Patienteninterfaces nach dem Stand der Technik (zahlenmäßig) deutlich weniger kritischer Parameter und einfacher Prozessschritte erlangt wird, da alle kritischen Parameter im einstückigen Linsenelement des Flüssigkeits-Patienteninterfaces konzentriert sind und somit nicht mehr verschiedene Teile des Flüssigkeits-Patienteninterfaces hochpräzise hergestellt und hochpräzise zueinander ausgerichtet werden müssen.

In einem erfindungsgemäßen Herstellungsverfahren für ein spezielles Flüssigkeits-Patienteninterface zur Fixierung der relativen geometrischen Lage eines Patientenauges zu einem Laser-Applikator eines ophthalmologischen Lasertherapiesystems, wird zunächst ein Linsenelement, das eine optische Zone, die eine Linsenfunktion aufweist, und einen an die optische Zone anschließenden Mantelbereich mit einem oberen Rand enthält, einstückig, bevorzugt in einem Spritzgussverfahren aus einem Polymer, gefertigt.

Das gefertigte Linsenelement wird hernach in ein einteiliges Konuselement, das eine Konuswandung, eine untere Sauglippe, eine Vakuum-Durchführung, und einen Einfüllkanal für Flüssigkeiten umfasst, eingelegt und verklebt.

Erfindungsgemäß wird dabei der obere Rand des Linsenelements so gefertigt, dass er eine direkte Verbindung zum Laser-Applikator ermöglicht. Dazu wird bevorzugt am oberen Rand des Linsenelements eine Struktur ausgebildet, die eine mechanisch stabile, direkte Verbindung mit einer Applikator-Schnittstelle des Laser-Applikators ermöglicht.

Im erfindungsgemäßen Herstellungsverfahren für ein Flüssigkeits-Patienteninterface, das besonders einfach ist, wenn ein Spritzgussverfahren zur Herstellung des Linsenelements eingesetzt wird, ist also in einem prinzipiell kurzem Verfahren nur auf wenige kritische Parameter zu achten.

Die vorliegende Erfindung soll nun anhand von Ausführungsbeispielen erläutert werden. Es zeigt:
- die Fig. 1a bis 1c ein Linsenelement 2 eines ersten Ausführungsbeispiels des erfindungsgemäßen Flüssigkeits-Patienteninterfaces in verschiedenen Ansichten;
- die Fig. 2a bis 2c einen Querschnitt durch eine Applikator-Schnittstelle eines Laser-Applikators eines ophthalmologischen Lasertherapiesystems sowie durch das erste Ausführungsbeispiel eines erfindungsgemäßen Flüssigkeits-Patienteninterfaces,
- die Fig. 3 eine Ansicht eines zweiten Ausführungsbeispiels des erfindungsgemäßen Flüssigkeits-Patienteninterfaces, und
- die Fig. 4 ein ophthalmologisches Lasertherapiesystem mit einem fixierten erfindungsgemäßen Flüssigkeits-Patienteninterface.

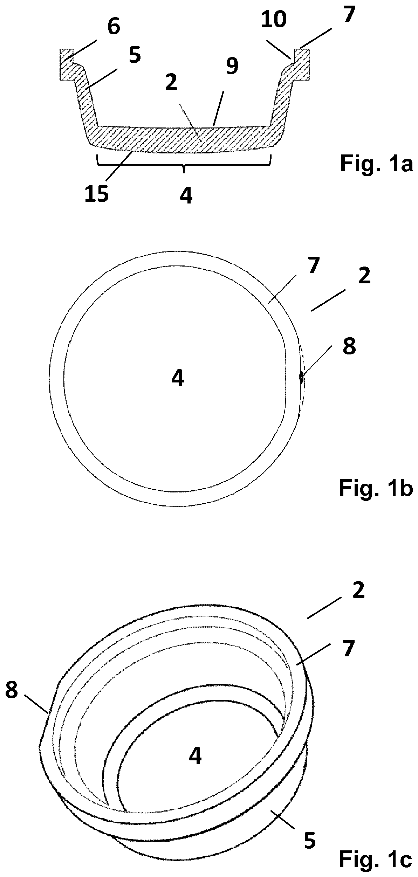

In den Fig. 1a bis 1c ist ein Linsenelement 2 eines ersten Ausführungsbeispiels des erfindungsgemäßen Flüssigkeits-Patienteninterfaces 1 in verschiedenen Ansichten dargestellt: in einer Schnittansicht von der Seite (Fig. 1a), in einer Draufsicht auf Stirnfläche 7 des oberen Randes 6 des Linsenelements 2 (Fig. 1b) und in einer Projektionsansicht (Fig. 1c). In diesem ersten Ausführungsbeispiel ist das Linsenelement 2 einstückig aus einem Polymer, konkret aus Polycarbonat, in einem Spritzgussverfahren gefertigt. In einem Spritzgussverfahren wird eine Form von einer Einfüllöffnung aus ausgegossen. Dabei entsteht ein Angusspunkt 8, üblicherweise in der Form eines Zapfens. Das Linsenelement 2 weist eine optische Zone 4 auf, an die sich ein Mantelbereich 5 anschließt. Dieser Mantelbereich 5 weist eine definierte Höhe h auf, der den Abstand der optischen Zone 4 des Linsenelements 2 von einem letzten optischen Applikator-Element 22 eines in dieser Figur nicht gezeigten Laser-Applikators 220 eines ophthalmologischen Lasertherapiesystems bestimmt. Der obere Rand 6 des Mantelbereichs 5 weist eine glatte (und hierzu polierte) Stirnfläche 7 für eine Vakuum-Ansaugung an eine Applikator-Schnittstelle 21 des Laser-Applikators 220 und einen Absatz 10 zur lateralen Ausrichtung bzgl. der Applikator-Schnittstelle 21 auf. Die optische Zone 4 weist eine Linsenfunktion 15 auf. Um im Einsatz am ophthalmologischen Lasertherapiesystem 100 Reflexionen am Linsenelement 2 und damit eine Rückspiegelung von Untersuchungsstrahlung eines OCT-Lasers und insbesondere von hochenergetischer Therapielaserstrahlung in den Laser-Applikator 220 des ophthalmologischen Lasertherapiesystems 100 zu vermeiden, weist das Linsenelement 2 an seiner oberen Begrenzungsfläche eine Antireflexschicht 9 auf.

In dem in der Fig. 1 gezeigten ersten Ausführungsbeispiel ist nun der Angusspunkt 8 des Linsenelements 2 - wie in der Draufsicht auf das Linsenelement 2 in der Fig. 1b dargestellt, am äußeren oberen Rand 6 angeordnet. Hierzu ist der äußere obere Rand 6 des Linsenelements 2 im Bereich des Angusspunktes 8 abgeschrägt, und zwar derart, dass der zapfenförmige Angusspunkt 8 nicht über den maximalen Durchmesser dieses oberen Randes 6 hinausragt, wie mit der Strich-Punkt-Linie in der Fig. 1b dargestellt. Auch in der Projektionsansicht der Fig. 1c ist diese Abschrägung zu sehen. Wenn darauf geachtet wird, dass der Angusspunkt 8 nicht über den maximalen Durchmesser des oberen Randes 6 hinausragt, entstehen keine Spannungen durch Verformung des Linsenelements 2 bei einer Vereinigung von Linsenelement 2 und Konuselement 3 zu einem einteiligen Flüssigkeits-Patienteninterface 1. Ansonsten müsste das Linsenelement 2 in das Konuselement 3 mit Kraftaufwand hineingepresst werden, was durch die dabei entstehende Verformung die optischen Eigenschaften undefiniert ändern kann.

In den Fig. 2a bis 2c ist ein Querschnitt durch eine Applikator-Schnittstelle 21 eines Laser-Applikators 220 eines ophthalmologischen Lasertherapiesystems 100 sowie durch das erste Ausführungsbeispiel eines erfindungsgemäßen Flüssigkeits-Patienteninterfaces 1 gezeigt. In diesem Flüssigkeits-Patienteninterface 1, das in der Fig. 2b im ungekoppelten Zustand gegenüber der Fig. 2a einer Applikator-Schnittstelle 21 eines Laser-Applikators 220 eines ophthalmologischen Lasertherapiesystems 100 dargestellt ist, ist das Linsenelement 2 der Fig. 1a bis 1c in einem Konuselement 3 eingelegt und dauerhaft verbunden. Die Fig. 2c zeigt schließlich das Flüssigkeits-Patienteninterface 1 im gekoppelten Zustand an der Applikator-Schnittstelle 21.

Das Konuselement 3 weist eine Wandung 16 auf, in die das Linsenelement 2 auf einem Absatz 17 eingelegt und durch eine Klebung dauerhaft verbunden ist. Durch die Wandung 16 verläuft eine Vakuum-Durchführung 11 in die Sauglippe 14 für die Vakuum-Ansaugung des Flüssigkeits-Patienteninterfaces 1 an ein Patientenauge 50. Durch die Wandung 16 verläuft des Weiteren ein Einfüllkanal 12 zum Befüllen des Volumens zwischen dem Linsenelement 2, der Wandung 16 des Konuselements 3 und einem Patientenauge 50 mit einer brechungsindex-adaptierten Flüssigkeit (z.B. einer Salzlösung, BSS) nach einer Vakuum-Ansaugung des Flüssigkeits-Patienteninterfaces 1 an das Patientenauge 50.

Die Wandung 16 des Konuselements 3 ist durch einen Kragen 13 verlängert. Dieser Kragen 13 legt sich im an der Applikator-Schnittstelle 21 angekoppelten Zustand um diese Applikator-Schnittstelle 21, um diese vom Patientenauge 50 abzuschirmen und so den Sterilbereich zu vergrößern. Allerdings liegt der Kragen 13 dann nicht direkt auf der Applikator-Schnittstelle 21 auf, sondern lässt einen Spalt 18, in der ein steriler Überzug (Drape) eingebracht werden kann, dass ebenfalls dem Schutz und der Ausweitung des Sterilbereichs dient.

Die Applikator-Schnittstelle 21 des Laser-Applikators 220 eines ophthalmologischen Lasertherapiesystems 100 weist einen Vakuumansaugkanal 23 auf, der bei Kopplung des Flüssigkeits-Patienteninterfaces 1 an die Applikator-Schnittstelle 21 auf der Stirnfläche 7 des oberen Randes 6 des Linsenelements 2 des Flüssigkeits-Patienteninterfaces 2 aufsetzt, und durch den dieses angesaugt wird. Zur korrekten lateralen Ausrichtung des Flüssigkeits-Patienteninterfaces 2 zur Applikator-Schnittstelle 21 vor der Vakuum-Ansaugung umfasst die Applikator-Schnittstelle 21 eine Zapfenstruktur 24. Diese greift in einen Absatz 10 im oberen Rand 6 des Linsenelements 2 des Flüssigkeits-Patienteninterfaces 1.

Die Applikator-Schnittstelle 21, die Teil eines Laser-Applikators 220 eines ophthalmologischen Lasertherapiesystems 100 ist, weist eine Laserapertur auf, durch die eine Therapielaserstrahlung in das Flüssigkeits-Patienteninterface 1 und von dort in das Auge 50 eines Patienten eintreten kann.

Die Applikator-Schnittstelle weist weiterhin ein letztes optisches Applikator-Element 22 auf, welches hier die letzte Linse des Applikator-Objektivs bildet, und Teil des optischen Systems zur Führung und Fokussierung einer Therapielaserstrahlung des Lasertherapiesystems 100, und ggf. auch eines optischen Systems zur Führung und Fokussierung einer Untersuchungsstrahlung, ist.

Die Fig. 3 zeigt eine Seitenansicht eines zweiten Ausführungsbeispiels des erfindungsgemäßen Flüssigkeits-Patienteninterfaces 1, in der eine bevorzugte Anordnung des Einfüllkanals 12 zur Vakuum-Durchführung 11 an der äußeren Wandung 16 des Konuselements 3 des Flüssigkeits-Patienteninterfaces 1 dargestellt ist. Die Vorteile einer solchen Anordnung wurden oben bereits beschrieben: Wie in den Fig. 2b und 2c ersichtlich, ist der Abstand zwischen dem Konuselement 3 und dem Linsenelement 2 für den Einfüllkanal 12 für Flüssigkeiten sehr gering. Um ein Einfüllen der brechungsindex-adaptierten Flüssigkeit zu ermöglichen, muss man hier mehr Platz zu gewinnen. Dazu wird, wie in diesem zweiten Ausführungsbeispiel des erfindungsgemäßen Flüssigkeits-Patienteninterfaces 1 ersichtlich, der Einfüllkanal 12 bevorzugt möglichst weit nach unten gezogen. Um nicht mit der Vakuum-Durchführung 11 konstruktiv zu kollidieren, können beide Kanäle, wie in der Fig. 3 gezeigt, gegeneinander versetzt werden. Sie liegen also nicht mehr vertikal in einer Linie (wie in der Fig. 2b und 2c dargestellt), sondern bevorzugt schräg zueinander.

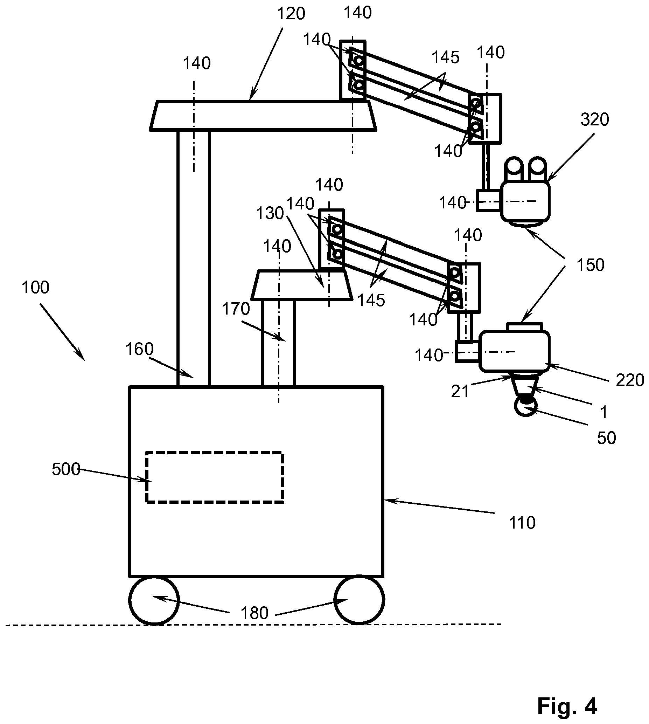

In der Fig. 4 ist ein ophthalmologisches Lasertherapiesystem 100 mit einem fixierten erfindungsgemäßen Flüssigkeits-Patienteninterface 1, das wiederum an einem Patientenauge 50 angedockt ist, in einer Seitenansicht dargestellt.

Dieses ophthalmologische Lasertherapiesystem 100 enthält einen Grundkörper 110, der auf einer Bewegungseinrichtung 180, die Rollen aufweist, gelagert ist. Aus diesem Grundkörper 110 ragen zwei als Achsen wirkende Stützstrukturen 160, 170 heraus, an denen jeweils ein Gelenkarm 120, 130 angeordnet ist. Der erste Gelenkarm 120 enthält ein Operationsmikroskop-Kopf 320, während am zweiten Gelenkarm 130 ein Laser-Applikator 220 angeordnet ist, aus dem im Betrieb eine fokussierte, gepulste Laserstrahlung, die in einer im Grundkörper 110 befindlichen (hier nicht gezeigten) Laserquelle - in diesem Fall einer Femtosekunden-Laserquelle - erzeugt wird und über eine Strahlführungsvorrichtung in Stützstruktur 170 und Gelenkarm 130 und eine Strahlfokussierungsoptik als Teil der Strahlführungsvorrichtung zum Laser-Applikator 220 geleitet wird. Beide Gelenkarme 120, 130 sind über ihre Gelenke 140 nahezu beliebig im Raum beweglich. Alle Komponenten dieses ophthalmologischen Untersuchungssystems 100 werden durch eine Steuervorrichtung 500 gesteuert.

Die beiden Gelenkarme 120, 130 können dadurch miteinander verbunden werden, dass zwei Teile einer Kopplungsstruktur 150, deren erster Teil am Operationsmikroskop-Kopf 320 des ersten Gelenkarms 120 angeordnet ist und deren zweiter Teil am Laser-Applikator 220 des zweiten Gelenkarms 130 angeordnet ist, miteinander in Verbindung gebracht werden. Damit das ophthalmologische Untersuchungs- und Therapiesystem 100 in jeder Position der Gelenkarme 120, 130 seine Stabilität behält und diese nicht etwa wegkippen, weisen die Gelenkarme 120, 130 Gewichtsausgleichstrukturen 145 auf.

Durch diese Gelenkarme 120, 130 hindurch wird auch ein Untersuchungsstrahl einer Vorrichtung für die optische Kohärenztomographie (OCT) geleitet. Dies ist in beiden Gelenkarmen 120, 130 mittels einer Lichtleitfaser möglich. Im Gelenkarm 130, durch den die gepulste Laserstrahlung der Therapielaservorrichtung zum Laser-Applikator-220 geleitet wird, ist dies alternativ möglich unter Nutzung der Strahlführungsvorrichtung mit ihrer Strahlfokussieroptik, die ansonsten von der gepulsten Laserstrahlung genutzt wird. In diesem Fall wird der Untersuchungsstrahl frei durch den Gelenkarm 130 hindurchgeleitet.

An einer Applikator-Schnittstelle 21 des Laser-Applikators 220 ist nun das erfindungsgemäße Flüssigkeits-Patienteninterface 1 mittels einer Vakuum-Ansaugung fixiert. Nach der Fixierung an der Applikator-Schnittstelle 21 wird das Flüssigkeits-Patienteninterface 1 mit seiner Sauglippe 14 auf dem Patientenauge 50 aufgesetzt und dort ebenfalls mit einer Vakuum-Ansaugung fixiert. Anschließend kann es mit einer Salzlösung, wie BSS, befüllt werden. Der Patient (nicht gezeigt), zu dem das hier gezeigte Patientenauge 50 gehört, liegt dabei bevorzugt auf einer Patientenliege neben dem ophthalmologischen Lasertherapiesystem 100.

Die Erfindung ist in den folgenden Ansprüchen definiert.

## Patentansprüche

1. Flüssigkeits-Patienteninterface (1) zur Fixierung der relativen geometrischen Lage eines Patientenauges (50) zu einem Laser-Applikator (220) eines ophthalmologischen Lasertherapiesystems (100), umfassend ein Linsenelement (2) und ein Konuselement (3), wobei das Linsenelement (2) in das Konuselement (3) eingelegt und dauerhaft so mit dem Konuselement (3) verbunden ist, dass das Flüssigkeits-Patienteninterface (1) einteilig ausgeführt ist, wobei
das Linsenelement (2) einstückig ausgebildet ist, eine optische Zone (4), die eine Linsenfunktion aufweist, und einen an die optische Zone (4) anschließenden Mantelbereich (5) mit einer definierten Höhe ungleich Null und mit einem oberen Rand (6) enthält, wobei der obere Rand (6) des Linsenelements (2) eine direkte Verbindung zum Laser-Applikator (220) ermöglicht,
das Flüssigkeits-Patienteninterface **gekennzeichnet dadurch, dass**
die Höhe des Mantelbereichs (5) des Linsenelements (2) direkt und allein den effektiven Abstand einer letzten Linse des Laser-Applikators (220) des ophthalmologischen Lasertherapiesystems (100) zur oberen Begrenzung bzw. Grenzfläche der optischen Zone (4) des Linsenelements (2) definiert.

2. Flüssigkeits-Patienteninterface (1) nach Anspruch 1, wobei der obere Rand (6) des Linsenelements (2) eine Struktur (7, 10) zur Ausbildung einer mechanisch stabilen, direkten Verbindung mit einer Applikator-Schnittstelle (21) des Laser-Applikators (220) des ophthalmologischen Lasertherapiesystems (100) aufweist, wobei die Struktur des oberen Randes (6) des Linsenelements (2) entweder
- eine Stirnfläche (7) zur Ausbildung einer vakuumdichten Verbindung mittels Vakuum-Ansaugung zu der Applikator-Schnittstelle (21) umfasst, wobei die Vakuum-Ansaugung entweder
= über die Evakuierung eines Volumens erfolgt, das begrenzt wird von einem letzten optischen Applikator-Element (22), der Applikator-Schnittstelle (21) und dem Linsenelement (2), wobei die Applikator-Schnittstelle (21) hierfür einen Vakuumansaugkanal (23) in das Volumen aufweist, oder
= über die Stirnfläche (7) erfolgt, wobei die Applikator-Schnittstelle (21) hierfür einen Vakuumansaugkanal (23) aufweist, der auf die Stirnfläche (7) aufsetzt; oder
- eine Struktur (10) aufweist, die eine formschlüssige oder formschlüssige und kraftschlüssige Verbindung mit der Applikator-Schnittstelle (21) ermöglicht.

3. Flüssigkeits-Patienteninterface (1) nach Anspruch 1 oder 2, wobei der obere Rand (6) des Linsenelements (2) eine positive Ausrichtstruktur aufweist, die eingerichtet ist, in eine an der Applikator-Schnittstelle (21) angeordnete negative Ausrichtstruktur (24) einzugreifen.

4. Flüssigkeits-Patienteninterface (1) nach einem der Ansprüche 1 bis 3, wobei das Linsenelement (2) aus einem Polymer, oder aus optischem Glas besteht.

5. Flüssigkeits-Patienteninterface (1) nach Anspruch 4, wobei das Linsenelement (2) aus einem Polymer durch ein Spritzgussverfahren ausgebildet ist, wobei der Angusspunkt (8) außerhalb der optischen Zone (4) angeordnet ist.

6. Flüssigkeits-Patienteninterface (1) nach Anspruch 5, wobei der Angusspunkt (8) entweder
- an einer Seite des oberen Randes (6) des Linsenelements (2), an dem dieses einen maximalen Durchmesser aufweist, angeordnet ist, wobei diese Seite derart abgeschrägt wird, dass ein beim Angießen entstehender Zapfen den maximalen Durchmesser des oberen Randes (6) nicht überschreitet, oder
- an der Innenseite des oberen Randes (6) oder an der Außenseite des Mantelbereiches (5) angeordnet ist.

7. Flüssigkeits-Patienteninterface (1) nach einem der Ansprüche 1 bis 6, wobei die untere Begrenzungsfläche (15) der optischen Zone (4) des Linsenelements (2) als optisches Element des Flüssigkeits-Patienteninterfaces (1) ausgebildet ist und insbesondere eine Linsenfunktion aufweist.

8. Flüssigkeits-Patienteninterface (1) nach einem der Ansprüche 1 bis 7, wobei die obere Begrenzungsfläche (9) der optischen Zone (4) des Linsenelements (2) eine Antireflexschicht (9) bzw. ein Antireflexschichtsystem aufweist.

9. Flüssigkeits-Patienteninterface (1) nach Anspruch 8, wobei die Antireflexschicht (9) bzw. das Antireflexschichtsystem ausgebildet ist, die Reflexion einer Strahlung in mindestens einem der folgenden Wellenlängenbereiche zu unterbinden, wobei ein Unterbinden in den jeweiligen Wellenlängenbereich mit einer zum Wellenlängenbereich angegebenen Reflexion R erfolgt:
- 1000nm bis 1100nm, R < 1%;
- 800nm bis 1200nm, R < 1%;
- 800nm bis 1000nm, R < 10%;
- 400nm bis 700nm, bei konstanter Reflexion R über diesen Wellenlängenbereich.

10. Flüssigkeits-Patienteninterface (1) nach einem der Ansprüche 1 bis 9, wobei
- das Linsenelement (2) eingerichtet ist, an einer Stirnseite (7) des oberen Randes (6) eine aus der Applikator-Schnittstelle (21) auskoppelnde Beleuchtung aufzunehmen, und/oder
- das Konuselement (3) eingerichtet ist, lokal einen direkten Kontakt zur Applikator-Schnittstelle (21) auszubilden und hierüber eine aus dem Laser-Applikator (220) auskoppelnde Beleuchtung aufzunehmen.

11. Flüssigkeits-Patienteninterface (1) nach einem der Ansprüche 1 bis 10, wobei das Konuselement (3) eine Konuswandung (16), eine untere Sauglippe (14), eine Vakuum-Durchführung (11), und einen Einfüllkanal (12) für Flüssigkeiten umfasst, wobei die Vakuum-Durchführung (11) durch die Konuswandung (16) in die Sauglippe (14) verläuft, und der Einfüllkanal (12) für Flüssigkeiten durch die Konuswandung (16) in ein bei Vakuum-Ansaugung von einem Patientenauge (50), der Konuswandung (16) und dem Linsenelement (2) gebildetes zweites Volumen verläuft.

12. Flüssigkeits-Patienteninterface (1) nach einem der Ansprüche 1 bis 11 wobei das Linsenelement (2) mit dem Konuselement (3) mit Hilfe eines Klebers, der beim Trocknungsvorgang keinerlei Zug- oder Verspannkräfte ausübt, verklebt ist.

13. Flüssigkeits-Patienteninterface (1) nach einem der Ansprüche 1 bis 12, wobei das Konuselement (3) einen Kragen (13) umfasst, der ausgebildet ist, die Konuswandung (16) zu verlängern.

14. Flüssigkeits-Patienteninterface nach einem der Ansprüche 11 bis 13 wobei der Einfüllkanal (12) so gegenüber der Vakuum-Durchführung (11) versetzt angeordnet ist, dass Einfüllkanal (12) und Vakuum-Durchführung (11) in einer Ansicht von oben auf das Flüssigkeits-Patienteninterface (1) nicht übereinander fallen, sondern in der Nähe zueinander, aber zueinander versetzt angeordnet sind.

15. Herstellungsverfahren für ein Flüssigkeits-Patienteninterface (1) zur Fixierung der relativen geometrischen Lage eines Patientenauges (50) zu einem Laser-Applikator (220) eines ophthalmologischen Lasertherapiesystems (100), bei dem
- ein Linsenelement (2), das eine optische Zone (4), die eine Linsenfunktion aufweist, und einen an die optische Zone (4) anschließenden Mantelbereich (5) mit einem oberen Rand (6) enthält, einstückig in einem Spritzgussverfahren aus einem Polymer gefertigt wird, und
- das Linsenelement (2) in ein einteiliges Konuselement (3), das eine Konuswandung (16), eine untere Sauglippe (14), eine Vakuum-Durchführung (11), und einen Einfüllkanal (12) für Flüssigkeiten umfasst, eingelegt und verklebt wird,
- wobei der obere Rand (6) des Linsenelements (2) so gefertigt wird, dass er eine direkte Verbindung des Linsenelements (2) zum Laser-Applikator (220) ermöglicht, wofür am oberen Rand (6) des Linsenelements (2) eine Struktur (7, 10) ausgebildet wird, die eine mechanisch stabile, direkte Verbindung mit einer Applikator-Schnittstelle (21) des Laser-Applikators (220) ermöglicht, und
- wobei die Höhe des Mantelbereichs (5) des Linsenelements (2) direkt und allein den effektiven Abstand der letzten Linse des Laser-Applikators (220) des ophthalmologischen Lasertherapiesystems (100) zur oberen Begrenzung bzw. Grenzfläche der optischen Zone (4) des Linsenelements (2) definiert.

## Claims

1. Liquid-patient interface (1) for fixing the relative geometric position of a patient's eye (50) with respect to a laser applicator (220) of an ophthalmological laser therapy system (100), said liquid-patient interface comprising a lens element (2) and a cone element (3), wherein the lens element (2) is inserted into the cone element (3) and permanently connected to said cone element (3) in such a way that the liquid-patient interface (1) has an integral configuration, wherein the lens element (2) is embodied in one piece and contains an optical zone (4), which has a lens function, and an envelope region (5), adjoining the optical zone (4), having a defined height not equal to zero and having an upper edge (6), wherein the upper edge (6) of the lens element (2) allows a direct connection to the laser applicator (220),
the liquid-patient interface being **characterized in that** the height of the envelope region (5) of the lens element (2) directly and solely defines the effective distance between a last lens of the laser applicator (220) of the ophthalmological laser therapy system (100) and the upper boundary respectively boundary surface of the optical zone (4) of the lens element (2).

2. Liquid-patient interface (1) according to Claim 1, wherein the upper edge (6) of the lens element (2) comprises a structure (7, 10) for forming a mechanically stable, direct connection to an applicator interface (21) of the laser applicator (220) of the ophthalmological laser therapy system (100), wherein the structure of the upper edge (6) of the lens element (2) either
- comprises an end face (7) for forming a vacuum-tight connection by way of vacuum suction onto the applicator interface (21), wherein the vacuum suction is implemented either
= by evacuating a volume that is delimited by a last optical applicator element (22), the applicator interface (21) and the lens element (2), the applicator interface (21) having a vacuum suction channel (23) into the volume to this end, or
= by way of the end face (7), the applicator interface (21) having a vacuum suction channel (23) that is positioned at the end face (7) to this end; or
- comprises a structure (10) that allows an interlocking or interlocking and force-fit connection to the applicator interface (21).

3. Liquid-patient interface (1) according to Claim 1 or 2, wherein the upper edge (6) of the lens element (2) comprises a positive alignment structure, which is configured to engage in a negative alignment structure (24) disposed at the applicator interface (21).

4. Liquid-patient interface (1) according to any one of Claims 1 to 3, wherein the lens element (2) consists of a polymer or an optical glass.

5. Liquid-patient interface (1) according to Claim 4, wherein the lens element (2) made of a polymer is formed by an injection moulding method, with the gate mark (8) being disposed outside of the optical zone (4).

6. Liquid-patient interface (1) according to Claim 5, wherein the gate mark (8) is disposed either
- at a side of the upper edge (6) of the lens element (2) where the latter has a maximum diameter, with this side being bevelled in such a way that a pin arising during moulding does not exceed the maximum diameter of the upper edge (6), or
- at the inner side of the upper edge (6) or at the outer side of the envelope region (5).

7. Liquid-patient interface (1) according to any one of Claims 1 to 6, wherein the lower delimiting face (15) of the optical zone (4) of the lens element (2) is embodied as an optical element of the liquid-patient interface (1) and, in particular, has a lens function.

8. Liquid-patient interface (1) according to any one of Claims 1 to 7, wherein the upper delimiting face (9) of the optical zone (4) of the lens element (2) has an antireflection layer (9) or an antireflection layer system.

9. Liquid-patient interface (1) according to Claim 8, wherein the antireflection layer (9) or the antireflection layer system is embodied to suppress the reflection of radiation in at least one of the following wavelength ranges, wherein a suppression in the respective wavelength range is implemented with a reflection R specified for the wavelength range:
- 1000 nm to 1100 nm, R < 1%;
- 800 nm to 1200 nm, R < 1%;
- 800 nm to 1000 nm, R < 10%;
- 400 nm to 700 nm, with a constant reflection R over this wavelength range.

10. Liquid-patient interface (1) according to any one of Claims 1 to 9, wherein
- the lens element (2) is configured to receive, at an end side (7) of the upper edge (6), illumination output coupling from the applicator interface (21), and/or
- the cone element (3) is configured to locally form a direct contact with the applicator interface (21) and thereby to receive illumination output coupling from the laser applicator (220).

11. Liquid-patient interface (1) according to any one of Claims 1 to 10, wherein the cone element (3) comprises a cone wall (16), a lower suction lip (14), a vacuum feedthrough (11), and a filling channel (12) for liquids, wherein the vacuum feedthrough (11) extends through the cone wall (16) into the suction lip (14) and the filling channel (12) for liquids extends through the cone wall (16) into a second volume formed by a patient's eye (50), the cone wall (16) and the lens element (2) in the case of vacuum suction.

12. Liquid-patient interface (1) according to any one of Claims 1 to 11, wherein the lens element (2) is adhesively bonded to the cone element (3) with the aid of an adhesive that exerts no tensile or warping forces during the drying process.

13. Liquid-patient interface (1) according to any one of Claims 1 to 12, wherein the cone element (3) comprises a collar (13) that is embodied to lengthen the cone wall (16) .

14. Liquid-patient interface according to any one of Claims 11 to 13, wherein the filling channel (12) is disposed with such an offset in relation to the vacuum feedthrough (11) that filling channel (12) and vacuum feedthrough (11) do not coincide but are arranged close to, but offset from, one another in a view on the liquid-patient interface (1) from above.

15. Production method for a liquid-patient interface (1) for fixing the relative geometric position of a patient's eye (50) with respect to a laser applicator (220) of an ophthalmological laser therapy system (100), wherein
- a lens element (2) containing an optical zone (4), which has a lens function, and an envelope region (5), adjoining the optical zone (4), having an upper edge (6) is manufactured in one piece from a polymer in an injection moulding method, and
- the lens element (2) is inserted into and adhesively bonded with an integral cone element (3), which comprises a cone wall (16), a lower suction lip (14), a vacuum feedthrough (11), and a filling channel (12) for liquids,
- wherein the upper edge (6) of the lens element (2) is manufactured such that it allows a direct connection of the lens element (2) to the laser applicator (220), for the purposes of which a structure (7, 10) is formed at the upper edge (6) of the lens element (2), said structure allowing a mechanically stable, direct connection to an applicator interface (21) of the laser applicator (220), and
- wherein the height of the envelope region (5) of the lens element (2) directly and solely defines the effective distance between the last lens of the laser applicator (220) of the ophthalmological laser therapy system (100) and the upper boundary respectively boundary surface of the optical zone (4) of the lens element (2).

## Revendications

1. Interface patient-liquide (1) destinée à fixer la position géométrique relative d'un œil de patient (50) par rapport à un applicateur laser (220) d'un système de thérapie ophtalmologique par laser (100), ladite interface comprenant un élément formant lentille (2) et un élément conique (3), l'élément formant lentille (2) étant inséré dans l'élément conique (3) et relié de manière permanente à l'élément conique (3) de manière à réaliser l'interface liquide-patient (1) monobloc, l'élément formant lentille (2) étant conçu d'une seule pièce et contenant une zone optique (4), qui a une fonction de lentille, et une zone d'enveloppe (5) qui est adjacente à la zone optique (4) et qui a une hauteur définie non nulle et est pourvue d'un bord supérieur (6), le bord supérieur (6) de l'élément formant lentille (2) permettant une liaison directe à l'applicateur laser (220), l'interface patient-liquide étant **caractérisée en ce que**
la hauteur de la zone d'enveloppe (5) de l'élément formant lentille (2) définit directement et à elle seule la distance effective d'une dernière lentille de l'applicateur laser (220) du système de thérapie ophtalmologique par laser (100) à la limite ou surface limite supérieure de la zone optique (4) de l'élément formant lentille (2).

2. Interface patient-liquide (1) selon la revendication 1, le bord supérieur (6) de l'élément formant lentille (2) comportant une structure (7, 10) destinée à former une liaison directe, mécaniquement stable, avec une interface d'applicateur (21) de l'applicateur laser (220) du système de thérapie ophtalmologique par laser (100), la structure du bord supérieur (6) de l'élément formant lentille (2) comprenant
- soit une surface frontale (7) destinée à former une liaison étanche sous vide par aspiration sous vide avec l'interface d'applicateur (21), l'aspiration sous vide étant effectuée
= soit par évacuation d'un volume qui est délimité par un dernier élément d'applicateur optique (22), l'interface d'applicateur (21) et l'élément formant lentille (2), l'interface d'applicateur (21) comportant pour cela un conduit d'aspiration sous vide (23) menant dans le volume,
= soit par le biais de la surface frontale (7), l'interface d'applicateur (21) comportant pour cela un conduit d'aspiration sous vide (23) qui prend appui sur la surface frontale (7) ;
- soit une structure (10) qui permet d'établir une liaison par complémentarité de formes ou une liaison par complémentarité de formes et en force avec l'interface d'applicateur (21).

3. Interface patient-liquide (1) selon la revendication 1 ou 2, le bord supérieur (6) de l'élément formant lentille (2) comportant une structure d'orientation positive qui est conçue pour s'engager dans une structure d'orientation négative (24) disposée au niveau de l'interface d'applicateur (21).

4. Interface patient-liquide (1) selon l'une des revendications 1 à 3, l'élément formant lentille (2) comprenant un polymère ou un verre optique.

5. Interface patient-liquide (1) selon la revendication 4, l'élément formant lentille (2) en polymère étant formé par un processus de moulage par injection, le point d'injection (8) étant disposé à l'extérieur de la zone optique (4).

6. Interface patient-liquide (1) selon la revendication 5, le point d'injection (8) étant disposé
- soit sur un côté du bord supérieur (6) de l'élément formant lentille (2) où celui-ci a un diamètre maximal, ce côté étant biseauté de manière à ce qu'un téton formé lors de l'injection ne dépasse pas le diamètre maximal du bord supérieur (6),
- soit du côté intérieur du bord supérieur (6) ou du côté extérieur de la zone d'enveloppe (5).

7. Interface patient-liquide (1) selon l'une des revendications 1 à 6, la surface de délimitation inférieure (15) de la zone optique (4) de l'élément formant lentille (2) étant conçue comme un élément optique de l'interface patient-liquide (1) et présentant en particulier une fonction de lentille.

8. Interface patient-liquide (1) selon l'une des revendications 1 à 7, la surface limite supérieure (9) de la zone optique (4) de l'élément formant lentille (2) comportant une couche anti-réflectrice (9) ou un système de couches anti-réflectrices.

9. Interface patient-liquide (1) selon la revendication 8, la couche anti-réflectrice (9) ou le système de couches anti-réflectrices étant conçu(e) pour supprimer la réflexion d'un rayonnement dans au moins l'une des plages de longueurs d'onde suivantes, la suppression dans la plage de longueurs d'onde respective étant effectuée avec une réflexion R spécifiée pour la plage de longueurs d'onde :
- 1000 nm à 1100 nm, R < 1 % ;
- 800 nm à 1200 nm, R < 1 % ;
- 800 nm à 1000 nm, R < 10 % ;
- 400 nm à 700 nm, avec une réflexion R constante dans cette plage de longueurs d'onde.

10. Interface patient-liquide (1) selon l'une des revendications 1 à 9,
- la lentille (2) étant conçue pour recevoir un éclairage sortant par couplage de l'interface d'applicateur (21) au niveau d'une surface frontale (7) du bord supérieur (6), et/ou
- l'élément conique (3) étant conçu pour former localement un contact direct avec l'interface d'applicateur (21) et recevoir un éclairage sortant par couplage de l'applicateur laser (220).

11. Interface patient-liquide (1) selon l'une quelconque des revendications 1 à 10, l'élément conique (3) comprenant une paroi conique (16), une lèvre d'aspiration inférieure (14), un passage de vide (11) et un conduit de remplissage (12) destiné à des liquides, le passage de vide (11) s'étendant à travers la paroi conique (16) jusque dans la lèvre d'aspiration (14), et le conduit de remplissage (12) destiné à des liquides s'étendant à travers la paroi conique (16) jusque dans un deuxième volume formé par un œil de patient (50), la paroi conique (16) et l'élément formant lentille (2) lors de l'aspiration sous vide.

12. Interface patient-liquide (1) selon l'une des revendications 1 à 11, l'élément formant lentille (2) étant collé à l'élément conique (3) à l'aide d'un adhésif qui n'exerce aucune force de traction ou de serrage pendant le processus de séchage.

13. Interface patient-liquide (1) selon l'une des revendications 1 à 12, l'élément conique (3) comprenant une collerette (13) qui est conçue pour prolonger la paroi conique (16).

14. Interface patient-liquide selon l'une des revendications 11 à 13, le conduit de remplissage (12) étant disposé de manière décalée par rapport au passage de vide (11) de manière que le conduit de remplissage (12) et le passage de vide (11) ne se chevauchent pas dans une vue de dessus de l'interface patient-liquide (1), mais soient disposés à proximité l'un de l'autre tout en étant décalés l'un de l'autre.

15. Procédé de fabrication d'une interface patient-liquide (1) destinée à fixer la position géométrique relative d'un œil de patient (50) par rapport à un applicateur laser (220) d'un système de thérapie ophtalmologique par laser (100), procédé dans lequel
- un élément formant lentille (2) qui contient une zone optique (4), qui présente une fonction de lentille, et une zone d'enveloppe (5) qui est adjacente à la zone optique (4) et qui est pourvue d'un bord supérieur (6), est fabriqué d'une seule pièce en polymère dans un procédé de moulage par injection, et
- l'élément formant lentille (2) est inséré et collé dans un élément conique (3) monobloc qui comprend une paroi conique (16), une lèvre d'aspiration inférieure (14), un passage de vide (11) et un conduit de remplissage (12) destiné à des liquides,
- le bord supérieur (6) de l'élément formant lentille (2) étant fabriqué de manière à permettre une liaison directe de l'élément formant lentille (2) à l'applicateur laser (220), ce pour quoi une structure (7, 10) est formée au niveau du bord supérieur (6) de l'élément formant lentille (2), laquelle structure permet une liaison directe, mécaniquement stable, à une interface d'applicateur (21) de l'applicateur laser (220), et
- la hauteur de la zone d'enveloppe (5) de l'élément formant lentille (2) définissant directement et à elle seule la distance effective de la dernière lentille de l'applicateur laser (220) du système de thérapie ophtalmologique par laser (100) à la limite ou surface limite supérieure de la zone optique (4) de l'élément formant lentille (2).
